# EUROPEAN PATENT APPLICATION

(11) **EP 3 782 478 A1**
(43) Date of publication of application: **24.02.2021**
(21) Application number: 19193194.8
(22) Date of filing: 22.08.2019
(51) Int. Cl.: A23L 21/10, C12P 7/56, A23L 29/00, A23L 31/00

(54) **FOOD, FOOD PRECURSORS OR BEVERAGES COMPRISING D-LACTIC ACID AND/OR A SALT THEREOF AND A METHOD OF PRODUCING THE SAME**

(71) Applicant: BrainBoost Solutions, 6363 Fürigen (CH)
(72) Inventor: Friedrichson, Tim, 01309 Dresden (DE); Festel, Gunter, 6363 Fürigen (CH)
(74) Representative: Stolmár & Partner Patentanwälte PartG mbB

(57) **Abstract**

The present invention relates to food, food precursors or beverages comprising D-lactic acid and/or a salt thereof and a method for producing the same. Further, the present invention relates to the use of lactic acid bacteria in a method for producing food, food precursors or beverages comprising D-lactic acid and/or a salt thereof and to lactic acid bacteria strains for use in this method. The present invention is based on the finding that D-lactic acid and/or a salt thereof can protect mitochondria against intrinsic (e.g. genetic mutation) or extrinsic (e.g. mitochondrial toxins, pesticides) factors that lead to mitochondrial dysfunction, whereas L-lactic acid is not capable to support, maintain or reestablish mitochondrial function.

## Description

The present invention relates to food, food precursors or beverages comprising D-lactic acid and/or a salt thereof and a method for producing the same. Further, the present invention relates to the use of lactic acid bacteria in a method for producing food, food precursors or beverages comprising D-lactic acid and/or a salt thereof and to a lactic acid bacteria strain for use in this method.

Lactic acid is a chiral alpha-hydroxy acid consisting of two optical isomers, L-(+)-lactic acid or (S)-lactic acid and D-(-)-lactic acid or (R)-lactic acid. Both isomers differ in their metabolism, tissue and plasma concentration and biological function. Whereas L-lactic acid historically has been regarded as the desirable, physiologically active and metabolic energy providing isomer, D-lactic acid has long been considered to be metabolically inert.

Lactic acid is widely used in plastic industry, food and drug industry, and cosmetic industry. Most of the industrial production of lactic acid is achieved by fermentation of carbohydrates such as glucose, sucrose, maltose, starch or cellulose, wherein microorganism capable of producing lactic acid are bacteria and fungi. At present, L-lactic acid is widely produced in an industrial scale.

Lactic acid has the IUPAC name 2-hydroxypropanoic acid and the molecular formula C₃H₆O₃. Lactic acid is found primarily in sour milk products, such as koumiss, laban, yogurt, buttermilk, kefir, some cottage cheeses and kombucham but also, for example, in pickled vegetables, and cured meats and fish. Both optical isomers of lactic occur naturally in fermented foods. For example, WO 95/22911 discloses an alcohol-free refreshing drink produced organically from pure natural products.

Lactic acid and a salt thereof are approved food additives in the EU, US, Australia, New Zealand and Japan. Lactic acid is furthermore listed by its INS number 270 or as E number E270. Lactic acid is used in the art as a food preservative, curing agent, and flavoring agent. It is used in processed foods and as a decontaminant during meat processing. The amount of L- and D-lactic acid that can be added to food products is generally without limitations, except for those imposed by good manufacturing practice.

The regular amount of lactic acid is, for example, for yoghurt about 0.8% (w/w), and for buttermilk 1.5% (w/w), wherein the majority of lactic acid is L-lactic acid. High levels of D-lactic acid are found in fermented milk products such as yoghurt and cheese.

However, in case of yoghurt made with strains of *Lactobacillus bulgaricus* or *Lactobacillus acidophilus* about 90% of lactic acid is D-lactic acid.

The present invention is based on the finding that D-lactic acid and/or a salt thereof can protect mitochondria against intrinsic (e.g. genetic mutation) or extrinsic (e.g. mitochondrial toxins, pesticides) factors that lead to mitochondrial dysfunction. Surprisingly, L-lactic acid was not capable to support, maintain or reestablish mitochondrial function.

WO 2015/150383 discloses D-lactic acid for use in the treatment of neurodegenerative diseases being associated with a decline in mitochondrial activity selected from the group consisting of Parkinson's disease, Alzheimer's disease, Huntington's disease, Amyotrophic lateral diseases and other neurodegenerative diseases.

In accordance with the present invention, food, food precursors or beverages comprising D-lactic acid and/or a salt thereof and a method of producing the same are provided in order to support, maintain and reestablish mitochondrial function in a human.

Towards this end, using D-lactic acid producing bacteria, conditions have been established to produce high concentrations of D-lactic acid by fermentation. Hence, the formulation of the invention comprises more D-lactic acid and/or a salt thereof than the amount of D-lactic acid found in natural food. D-lactic acid is a hydroxycarboxylic acid dissociating under physiological conditions, whereby the salt thereof is commonly referred as lactate.

The objective of the present invention is therefore to provide food, food precursors or beverages comprising D-lactic acid and/or a salt thereof and a method for producing the same. The present invention further provides use of lactic acid bacteria in a method for producing food, food precursors or beverages comprising D-lactic acid and/or a salt thereof and a lactic acid bacteria strain for use in this method.

The method for producing food or food precursors comprising D-lactic acid and/or a salt thereof according to the present invention comprises the steps of:
a) providing a first substrate;
b) treating the first substrate with microorganisms.

In accordance with a preferred embodiment of the invention, the first substrate comprises fungi, algae, molasses, botanicals, plants, fruits, all monosaccharide-containing botanicals commonly used in human and animal nutrition, and processed products of the before mentioned substrates, comprising juices, pulps, extracts, blends thereof.

Optionally, enzymatic hydrolysis of carbohydrates contained in the first substrate can be performed, for example using amylase, cellulase, pectinase, peptidase or xylanase in order to increase the concentration of readily fermentable monosaccharides.

In an embodiment of the invention, the microorganism for treating the first substrate is a lactic acid bacterium, that completely, predominantly or partly produces D-lactic acid. Homofermentative lactic acid bacteria strains, for example *Lactobacillus, Streptococcus, Enterococcus, Lactococcus, Pediococcus,* are preferred, because of their ability to convert hexoses (i.e. glucose) as a substrate into two lactic acid molecules without byproducts. In contrary, heterofermentative lactic acid bacteria strains, for example *Leuconostoc,* convert pentoses or hexoses to lactic acid and byproducts, such as ethanol and acetic acid.

In a more preferred embodiment, the lactic acid bacterium is selected from the group of *Lactobacillus coryniformis, Lactobacillus jensenii, Lactobacillus delbrueckii, Sporolactobacillus inulinus.*

Treating of the first substrate with microorganism according to the present invention relates to fermentation, i.e. the microbial or enzymatic conversion of organic substances into acid by addition of bacterial, fungal or other biological cell cultures or by the addition of enzymes. Treating of the first substrate may comprise anaerobic fermentation, oxidative fermentation, conversion of organic materials due to microbial or enzymatic processes with or without involvement of oxygen (bioconversion and biocatalysis), controlled production of biotic metabolites with or without involvement of oxygen, submersfermentation, solid phase-fermentation on a carrier material, batch fermentation, fed-batch fermentation, continuous fermentation.

In a further embodiment, the method for producing food or food precursors comprising D-lactic acid further comprises the steps of:
c) providing a second substrate, and
d) mixing the first substrate obtained in step (b) with the second substrate.

Mixing of the first substrate obtained in step (b) with the second substrate comprises mixing, stirring or kneading.

In an embodiment, the second substrate comprises fungi, algae, molasses, botanicals, plants, fruits, all monosaccharide-containing botanicals commonly used in human and animal nutrition, and processed products of the before mentioned substrates, comprising juices, pulps, extracts, blends thereof.

In another embodiment, steps c) and d) can be repeated during the fermentation. Substrates differing from each other can be added.

In a further embodiment, substrates are added continuously and final products are removed continuously.

Optionally, enzymatic hydrolysis of carbohydrates contained in the second substrate or in subsequent added substrates can be performed, for example using cellulase, pectinase, peptidase or xylanase in order to increase the concentration of readily fermentable monosaccharides.

In another embodiment of the present invention, the method comprises the step of treating the second substrate with microorganisms and/or treating the mixture obtained in step d) with microorganism.

In another embodiment of the present invention, the method comprises the step of treating the substrate added during repetition of steps c) and d) with microorganisms and/or treating the mixture obtained after repetition of step c) and d) with microorganism

In an embodiment of the invention, the microorganism for treating the second substrate and/or treating the mixture obtained in step d) with microorganism is a lactic acid bacterium, that completely, predominantly or partly produces D-lactic acid. Homofermentative lactic acid bacteria strains, for example *Lactobacillus, Streptococcus, Enterococcus, Lactococcus, Pediococcus,* are preferred, because of their ability to convert hexoses (i.e. glucose) as a substrate to two lactic acid molecules without byproducts.

In contrary, heterofermentative lactic acid bacteria strains, for example *Leuconostoc,* convert pentoses or hexoses to lactic acid and byproducts, such as ethanol and acetic acid.

In a more preferred embodiment, the lactic acid bacterium is selected from the group of *Lactobacillus coryniformis, Lactobacillus jensenii, Lactobacillus delbrueckii, Sporolactobacillus inulinus.*

Treating of the second substrate with microorganism according to the present invention relates to fermentation, i.e. the microbial or enzymatic conversion of organic substances into acid, gases or alcohol by addition of bacterial, fungal or other biological cell cultures or by the addition of enzymes. Treating of a second substrate may comprise anaerobic fermentation, oxidative fermentation, conversion of organic materials due to microbial or enzymatic processes with or without involvement of oxygen (bioconversion and biocatalysis), controlled production of biotic metabolites with or without involvement of oxygen, submersfermentation, solid phase-fermentation on a carrier material, batch fermentation, fed-batch fermentation, continuous fermentation.

In another embodiment, the method according to the present invention further comprises the step of:
e) processing the substrate obtained in steps (b) and/or (d) to a beverage.

Processing of the substrate obtained in steps (b) and/or (d) to a beverage comprises mixing, stirring, mashing, crushing, chopping, squeezing, extracting.

The product obtained in step b), d) or e) can remain untreated. In another embodiment, microorganism can be killed by methods known in the art after step b), d) or e). Another embodiment comprises the heat-killing of microorganism after step b), d) or e), preferably by pasteurization with subsequent cooling.

The method for producing food or food precursors comprising D-lactic acid further comprises the steps of direct addition of synthetic D-lactic acid and/or semi-synthetic D-lactic acid to the first and/or second substrate and/or to the substrates obtained in step c), d) and e).

Direct addition of D-lactic acid and/or semi-synthetic D-lactic acid to the first and/or second substrate or to the substrates obtained in step c), d) and e) comprises addition by mixing, stirring, kneading or other means of common techniques applied to production of food, food precursors and beverages.

The present invention provides food, food precursors or beverages prepared by a method described above.

According to another embodiment of the present invention, food, food precursors or beverages prepared by this method further comprise one or more selected from micronutrients, functional foods, food supplements or other compounds.

Micronutrients are essential elements required in small quantities throughout the life cycle to orchestrate a range of physiological functions to maintain health. For human nutrition, micronutrient requirements are in amounts generally less than 100 milligrams per day. Micronutrients are vitamins, minerals (quantity elements and trace elements), proteinogenic amino acids and omega fatty acids, for example Vitamin A, B₁, B₂, B₃, B₆, B₁₂, C, D, E, Niacin, Coenzyme Q₁₀, Iodine, Iron, Selenium.

Functional food is food enriched with additional ingredients, for example vitamins, minerals, bacterial cultures and unsaturated fatty acids.

Food supplements are dietary supplements comprising concentrates of nutrients or other substances having a nutritional or physiological effect alone or in combination, which are intended to supplement the diet.

According to one embodiment of the present invention, food, food precursors or beverages comprising D-lactic acid and/or a salt thereof are in a liquid, semiliquid or solid form.

Food, food precursors or beverages prepared by the method according to the present invention comprise at least 0.8 % (w/w), preferably 2 % (w/w) up to 6 % (w/w) of D-lactic acid and/or a salt thereof.

Food, food precursors or beverages prepared by the method according to the present invention comprise according to another preferred embodiment at least 0.8 % (w/w), preferably 2 % (w/w) up to 6 % (w/w) of D-lactic acid and/or a salt thereof and in addition one or more selected from micronutrients, functional foods, food supplements or other compounds.

Further, the present invention provides the use of lactic acid bacteria in a method for producing food, food precursors or beverages comprising D-lactic acid and/or a salt thereof, preferably in a method according to the present invention.

In a preferred embodiment, the use of lactic acid bacterium comprises the use of lactic acid bacteria selected from the group *of Lactobacillus coryniformis, Lactobacillus jensenii, Lactobacillus delbrueckii, Sporolactobacillus inulinus.*

In another embodiment of the present invention, the method for producing food or food precursors comprising D-lactic acid and/or a salt thereof further comprises the steps of direct addition of synthetic D-lactic acid and/or semi-synthetic D-lactic acid and/or salts thereof to food, food precursors or beverages.

Addition of synthetic D-lactic acid and/or semi-synthetic D-lactic acid and/or salts thereof to food, food precursors or beverages comprises mixing, stirring, kneading or other means of common techniques applied to production of food, food precursors and beverages.

In a further aspect, the present invention provides food, food precursors or beverages comprising at least 0.8 % (w/w), preferably 2 % (w/w) up to 6 % (w/w) D-lactic acid and/or a salt thereof.

Food, food precursors or beverages comprising D-lactic acid and/or a salt thereof according to another embodiment, additionally comprise one or more selected from micronutrients, functional foods, food supplements or other compounds.

In another aspect, the present invention provides a lactic acid bacteria strain for use in a method for producing food, food precursors or beverages comprising D-lactic acid and/or a salt thereof, wherein the amount of D-lactic acid or a salt thereof is at least 0.8 % (w/w), preferably 2 % (w/w) up to 6 % (w/w).

The invention is described in more detail by way of the following figures and examples thereto, which show certain embodiments of the invention without being meant to be limiting for the scope of the invention.

### Figures

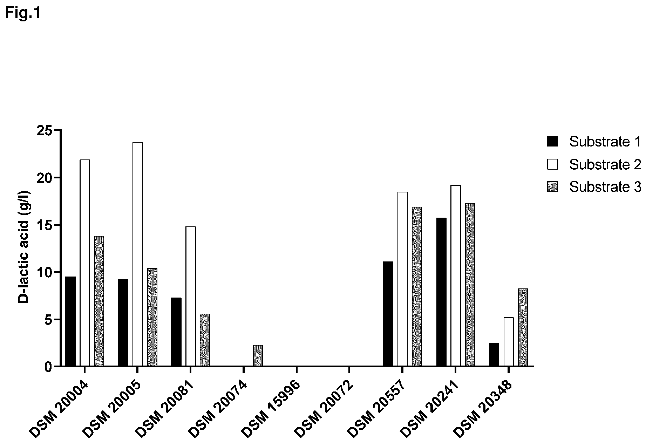

**Fig. 1** shows the enrichment of D-lactic acid of different bacterium strains and different substrates.

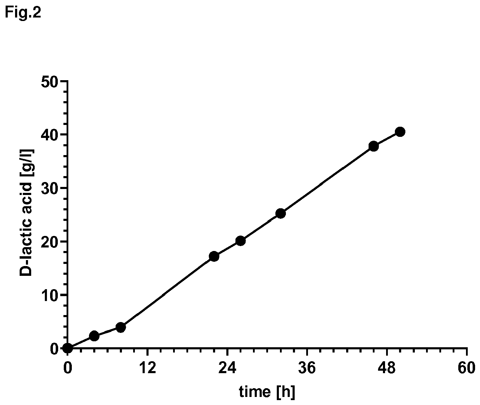

**Fig.2** shows the enrichment of D-lactic acid (increase of concentration [g/L]) during fermentation over a time period of 0 to 50 h as described in Example 2.

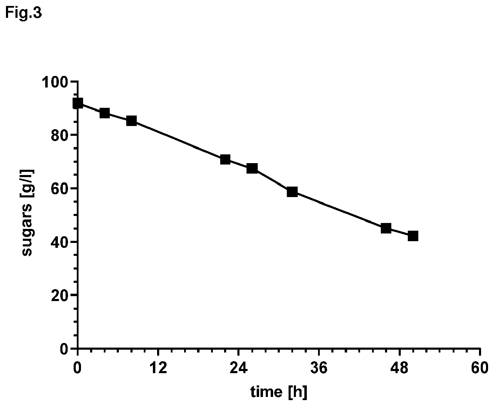

**Fig.3** shows the decrease of sugar concentration [g/L] during fermentation over a time period of 0 to 50 h as described in Example 2.

### Examples

### Example 1 - Evaluation of different bacterium strains and different substrates for the enrichment of D-lactic acid in food, food precursors or beverages

Frozen, whole mirabelles with pit were thawed completely. The mirabelles were pitted and the remaining skins and pulps were thoroughly blended. The pH of the blended mirabelle fruit masss was 3.2

Complex carbohydrates in the blended mirabelle fruit mass were optionally enzymatically hydrolyzed to increase the concentration of readily fermentable monosaccharides. Therefore, hydrolysis of mirabelle jam under use of different enzymes was performed as follows:

| | |
|---|---|
| Substrate 1: | 1 ml/kg Cellulase (Accellerase 1500) and 1.25 ml/kg Pektinase (Pektinase L-40) |
| Substrate 2: | 1 ml/kg Cellulase (Accellerase 1500), 1.25 ml/kg Pektinase (Pektinase L-40) and 2.5 g/L yeast extract in powdered form |
| Substrate 3: | 1 ml/kg Cellulase (Accellerase 1500), 1.25 ml/kg Pektinase (Pektinase L-40) and 0.25 g/kg Protease (Fermgen) |

Hydrolysis was performed for 24 h at pH 4.5 (adjusted with 20% NaOH) in shake flasks at 50°C and 150 rpm. After hydrolysis, the pH was adjusted to 6.5 with 20% NaOH.

A fermentation of the blended fruit mass treated as described above was performed with different D-lactic acid producing hetero- and homo-fermentative bacterial strains at their optimal temperature ranging from 30 to 52°C as follows:

| **Bacterial strain** | **DSMZ code** | **ATCC code** | **T [°C]** |
|---|---|---|---|
| *Bacillus coagulans* | DSM 2314 | n.a. | 52 |
| *Lactobacillus coryniformis subsp. torquens* | DSM 20004 | ATCC 25600 | 30 |
| *Lactobacillus coryniformis subsp. torquens* | DSM 20005 | n.a. | 30 |
| Lactobacillus delbrueckii subsp. bulgaricus | DSM 20081 | ATCC 11842 | 37 |
| *Lactobacillus delbrueckii subsp. delbrueckii* | DSM 20074 | ATCC 9649 | 37 |
| *Lactobacillus delbrueckii subsp. indicus* | DSM 15996 | n.a. | 37 |
| *Lactobacillus delbrueckii subsp. lactis* | DSM 20072 | ATCC 12315 | 37 |
| *Lactobacillus jensenii* | DSM 20557 | ATCC 25258 | 37 |
| *Leuconostoc mesenteroides subsp. mesenteroides* | DSM 20241 | ATCC 25600 | 30 |
| *Sporolactobacillus inulinus* | DSM 20348 | ATCC 15538 | 37 |

As preculture, each bacterial strain was inoculated into Man-Rogosa-Sharpe (MRS) medium and incubated 24 h at its optimal temperature. MRS medium is a liquid medium recommended for use in the cultivation of Lactobacillus species and lactic acid bacteria.

As substrates, 10 mL each of each hydrolysed mirabelle jam (substrate 1, 2 or 3) was filled into separate sterile glass tubes. For *Sporolactobacillus inulinus* (DSM 20348) each hydrolysed mirabelle jam (substrate 1, 2 or 3) was additionally supplemented with 60 g/l CaCO₃. After incubation time of 24 h, 100 µL preculture containing the respective bacterial strain was added to the 10 mL mirabelle jam into the glass tube and fermentation was performed at the optimal temperature of each respective bacterial strain for 48 h.

After 48 h of fermentation, D-lactic acid and L-lactic acid concentration, respectively, was determined enzymatically with a commercial D-lactic acid (D-lactate) and L-lactic acid (L-lactate) assay kit (Megazyme, Wicklow, Ireland). Several hetero- and homo-fermentative bacterial strains produce D-lactic acid in all three substrates. However, homo-fermentative strains are preferred according to this invention, because they exclusively synthesize lactic acid and no other byproducts, such as acetate or ethanol. Control (not containing bacteria) and negative control (using strain DSM 2314, exclusively producing L-lactic acid) do not produce D-lactic acid in any of the three substrates.

The resulting concentrations of glucose, fructose, saccharose and lactic acids after fermentation of 48 h are summarized below:
Substrate 1:

| DSMZ code | T [°C] | pH | glucose [g/L] | fructose [g/L] | sucrose [g/L] | lactic acid [g/L] |
|---|---|---|---|---|---|---|
| DSM 2314 L-lactic acid | 52 | 5.94 | 46.9 | 45.8 | 4.6 | 0 |
| DSM 20004 | 30 | 6.02 | 43.1 | 37.6 | 4.9 | 9.5 |
| DSM 20005 | 30 | 6.02 | 42.6 | 39.5 | 4.9 | 9.2 |
| DSM 20081 | 37 | 6.05 | 38.9 | 44.6 | 4.8 | 7.3 |
| DSM 20074 | 37 | 6.08 | 46.3 | 44.9 | 4.5 | 0 |
| DSM 15996 | 37 | 6.10 | 46.2 | 44.4 | 4.3 | 0 |
| DSM 20072 | 37 | 5.91 | 45.1 | 43.2 | 4.8 | 0 |
| DSM 20557 | 37 | 5.79 | 42.5 | 36.2 | 4.6 | 11.1 |
| DSM 20241 heterofermentative | 30 | 5.89 | 36.8 | 28.9 | 4.7 | 15.7 |
| DSM 20348 | 37 | 6.01 | 45.2 | 44.6 | 4.6 | 2.5 |
| control | 30 | 6.15 | 46.9 | 44.1 | 4.9 | 0 |

Substrate 2:

| DSMZ code | T [°C] | pH | glucose [g/L] | fructose [g/L] | sucrose [g/L] | lactic acid [g/L] |
|---|---|---|---|---|---|---|
| DSM 2314 L-lactic acid | 52 | 5.36 | 43.9 | 43.1 | 4.8 | 4.1 |
| DSM 20004 | 30 | 5.83 | 34.9 | 28.9 | 4.6 | 21.9 |
| DSM 20005 | 30 | 5.71 | 34.9 | 28.5 | 4.5 | 23.7 |
| DSM 20081 | 37 | 6.02 | 31.6 | 43.7 | 4.6 | 14.8 |
| DSM 20074 | 37 | 5.91 | 45.4 | 43.9 | 4.8 | 0 |
| DSM 15996 | 37 | 6.09 | 45.7 | 43.6 | 4.9 | 0 |
| DSM 20072 | 37 | 5.98 | 45.3 | 44.3 | 4.7 | 0 |
| DSM 20557 | 37 | 5.67 | 39.5 | 28.8 | 4.5 | 18.5 |
| DSM 20241 heterofermentative | 30 | 5.76 | 32.3 | 24.7 | 4.4 | 19.2 |
| DSM 20348 | 37 | 5.92 | 43.9 | 41.8 | 4.9 | 5.2 |
| control | 30 | 6.14 | 46.2 | 44.5 | 5.0 | 0 |

Substrate 3:

| DSMZ code | T [°C] | pH | glucose [g/L] | fructose [g/L] | sucrose [g/L] | lactic acid [g/L] |
|---|---|---|---|---|---|---|
| DSM 2314 L-lactic acid | 52 | 6.01 | 47.1 | 45.1 | 4.7 | 0 |
| DSM 20004 | 30 | 5.98 | 41.2 | 34.1 | 4.6 | 13.8 |
| DSM 20005 | 30 | 5.99 | 41.8 | 38.2 | 4.9 | 10.4 |
| DSM 20081 | 37 | 6.03 | 40.5 | 44.1 | 4.8 | 5.6 |
| DSM 20074 | 37 | 5.95 | 44.8 | 42.0 | 5.0 | 2.3 |
| DSM 15996 | 37 | 6.11 | 46.2 | 44.4 | 5.1 | 0 |
| DSM 20072 | 37 | 6.09 | 45.8 | 43.7 | 4.9 | 0 |
| DSM 20557 | 37 | 5.71 | 40.9 | 30.7 | 4.7 | 16.9 |
| DSM 20241 heterofermentative | 30 | 5.73 | 33.9 | 25.2 | 4.6 | 17.3 |
| DSM 20348 | 37 | 5.95 | 41.7 | 39.6 | 4.8 | 8.2 |
| control | 30 | 6.16 | 46.8 | 44.5 | 4.8 | 0 |

Strains DSM 20004 and DSM 20005 showed good yields of lactic acid in all tested substrates 1 to 3 having 9.5, 21.9, 13.8 g/L and 9.2, 23.7, 10.4 g/L lactic acid. Solely heterofermentative strain DSM 20241 showed even higher yields of 15.7 and 17.3 g/L in substrates 1 and 3.

Thus, *Lactobacillus coryniformis subsp. torquens* (DSM 20005) was used for producing mirabelle jam comprising D-lactic acid as shown in Example 2.

### Example 2 - Method for producing mirabelle jam comprising D-lactic acid

Frozen, whole mirabelles with pit were thawed completely. The mirabelles were pitted and the remaining skins and pulp were thoroughly blended to obtain a mirabelle jam. The pH of the blended Mirabelle jam was 3.2

As a preculture, Lactobacillus *coryniformis subsp. torquens* (DSM 20005) was inoculated into 150 mL MRS medium from three agar slant tubes and incubated 24 h at 30°C and 100 rpm. MRS medium is a liquid medium recommended for use in the cultivation of Lactobacillus species and lactic acid bacteria.

Complex carbohydrates in the blended mirabelle fruit mass are optionally enzymatically hydrolyzed to increase the concentration of readily fermentable monosaccharides. As a substrate, 2.5 kg mirabelle jam was adjusted to 2.5 l with sterile distilled water and transferred into an empty autoclaved 5-L-fermenter. For enzymatic hydrolysis, 2.5 mL Pektinase L-40 was added, pH was adjusted to 4.5 with 20% NaOH and hydrolysis was performed for 24 h at 50°C and a disk stirrer rotating at 500 rpm. After hydrolysis, the pH was adjusted to 6.0 with 20% NaOH.

After incubation time of 24 h, 150 mL preculture containing Lactobacillus *coryniformis subsp. tor*quens (DSM 20005) was added to the mirabelle jam into the 5-L-fermenter. Fermentation was performed for 50 h at 30°C and a blade disk stirrer rotating at 400 rpm. pH was monitored with a sensor and maintained at pH 6.0 by adding 20% NaOH through an automated pump system.

The resulting concentrations of D-lactic acid [g/L] after fermentation for 26 h and 50 h are 20.1 g/L and 40.5 g/L, respectively.

## Claims

1. Method for producing food or food precursors comprising D-lactic acid and/or a salt thereof, the method comprises the steps of:
a) providing a first substrate;
b) treating the first substrate with microorganisms.

2. The method according to claim 1, wherein the first substrate is fungi, algae, molasses, botanicals or processed products thereof.

3. The method according to any one of the preceding claims, wherein the microorganism is a lactic acid bacterium selected from the group of *Lactobacillus coryniformis, Lactobacillus jensenii, Lactobacillus delbrueckii, Sporolactobacillus inulinus.*

4. The method according to any one of the preceding claims, wherein the method further comprises the steps of:
c) providing a second substrate, and
d) mixing the first substrate obtained in step (b) with the second substrate.

5. The method according to any one of the preceding claims, wherein the method further comprises the step of:
e) processing the substrate obtained in steps (b) and/or (d) to a beverage.

6. Food, food precursors or beverages comprising D-lactic acid or a salt thereof prepared by a method according to any one of claims 1 to 5.

7. Food, food precursors or beverages according to claim 6, with one or more selected from micronutrients, functional foods, food supplements or other compounds.

8. Food, food precursors or beverages according to any one of the preceding claims, wherein the food, food precursors or beverages are in a liquid, semiliquid or solid form.

9. Food, food precursors or beverages according to any one of the preceding claims, wherein the amount of D-lactic acid and/or a salt thereof is at least 0.8 % (w/w), preferably 2 % (w/w) up to 6 % (w/w).

10. Use of lactic acid bacteria in a method for producing food, food precursors or beverages comprising D-lactic acid and/or a salt thereof, preferably in a method according to any one of claims 1 to 5.

11. Food, food precursors or beverages, comprising D-lactic acid and/or a salt thereof, wherein the amount of D-lactic acid is at least 0.8 % (w/w), preferably 2 % (w/w) up to 6 % (w/w).

12. Food, food precursors or beverages according to claim 11, with one or more selected from micronutrients, functional foods, food supplements or other compounds.

13. Lactic acid bacteria strain for use in a method for producing food, food precursors or beverages comprising D-lactic acid and/or a salt thereof, wherein the amount of D-lactic acid and/or a salt thereof is at least 0.8 % (w/w), preferably 2 % (w/w) up to 6 % (w/w).
